# EUROPÄISCHE PATENTSCHRIFT

(11) **EP 0 952 837 B1**
(45) Veröffentlichungstag und Bekanntmachung des Hinweises auf die Patenterteilung: **18.09.2002**
(21) Anmeldenummer: 97951916.2
(22) Anmeldetag: 19.11.1997
(51) Int. Cl.: A61K 31/70, A61K 31/715, A61K 31/575, A61P 3/06

(54) **VERWENDUNG VON WIRKSTOFFMISCHUNGEN, ENTHALTEND PHYTOSTENOLE UND/ODER PHYTOSTENOLESTER UND POTENZIERUNGSMITTEL ZUR HERSTELLUNG VON HYPOCHOLESTERINÄMISCHEN MITTELN**
USE OF MIXTURES OF ACTIVE SUBSTANCES, CONTAINING PHYTOSTENOLS AND/OR PHYTOSTENOL ESTERS AND POTENTIATORS, FOR THE PRODUCTION OF HYPOCHOLESTEREMIC AGENTS
UTILISATION DE MELANGES DE PRINCIPES ACTIFS, CONTENANT DES PHYTOSTENOLS ET/OU DES ESTERS DE PHYTOSTENOL ET DES AGENTS DE POTENTIALISATION, POUR LA PRODUCTION D'AGENTS HYPOCHOLESTEROLEMIANTS

(30) Priorität: 28.11.1996 DE 19649286; 13.01.1997 DE 19700796
(43) Veröffentlichungstag der Anmeldung: 03.11.1999
(73) Patentinhaber: Cognis Deutschland GmbH & Co. KG, 40589 Düsseldorf (DE)
(72) Erfinder: WEITKEMPER, Norbert, D-51375 Leverkusen (DE); FABRY, Bernd, D-41352 Korschenbroich (DE)
(86) Internationale Anmeldenummer: EP9706450
(87) Internationale Veröffentlichungsnummer: WO98023277

(56) Entgegenhaltungen:
- WO-A-94/01413

## Beschreibung

### Gebiet der Erfindung

Die Erfindung betrifft die Verwendung von Mischungen aus Phytostenolen bzw. Phytostenolestem und ausgewählten Potenzierungsmitteln zur Herstellung von Mitteln zur Verminderung des Cholesteringehaltes im Serum von Warmblütern.

### Stand der Technik

Unter hypochloesterinämischen Wirkstoffen werden Mittel verstanden, die zu einer Verminderung des Cholesteringehaltes im Serum von Warmblütern führen, ohne daß dadurch eine Hemmung oder Verringerung der Bildung von Cholesterin im Blut eintritt. Für diesen Zweck wurden bereits von Peterson et al. in **J.Nutrit. 50, 191 (1953)** Phytostenole, also pflanzliche Stenole, und deren Ester mit Fettsäuren vorgeschlagen. In die gleiche Richtung weisen auch die Patentschriften **US 3,089,939, US 3,203,862** sowie die deutsche Offenlegungsschrift **DE-OS 2035069** (Procter & Gamble). Die Wirkstoffe werden üblicherweise Brat- oder Speiseölen zugesetzt und dann über die Nahrung aufgenommen, wobei die Einsatzmengen jedoch in der Regel gering sind und üblicherweise unter 0,5 Gew.-% liegen, um zu verhindern, daß die Speiseöle eintrüben oder die Stenole bei Zusatz von Wasser ausgefällt werden. Für den Einsatz im Nahrungsmittelbereich, in Kosmetika, pharmazeutischen Zubereitungen und im Agrarsektor werden in der europäischen Patentanmeldung **EP-A1 0289636** (Ashai) lagerstabile Emulsionen der Stenolester in Zucker- oder Polyglycerinestem vorgeschlagen. Die Einarbeitung von Sitostanolestern zur Verminderung des Blutcholesteringehaltes in Margarine, Butter, Mayonnaise, Salatsaucen und dergleichen wird in der Europäischen Patentschrift **EP-B1 0594612** (Raision) vorgeschlagen.

Von Nachteil ist jedoch, daß die Phytostenolester den Nahrungsmitteln üblicherweise nur in geringen Mengen zugesetzt werden können, da ansonsten die Gefahr besteht, daß sie den Geschmack und/oder die Konsistenz der Mittel beeinträchtigen. Zur nachhaltigen Beeinflussung des Cholesteringehaltes im Blut wäre jedoch die Aufnahme größerer Mengen Phytostenole bzw. Phytostenolester wünschenswert. Weiterhin verbesserungswürdig ist die Geschwindigkeit, mit der die Stoffe den Gehalt an Cholesterin im Serum vermindern. Die Aufgabe der Erfindung hat folglich darin bestanden, diesen Mängeln abzuhelfen.

### Beschreibung der Erfindung

Gegenstand der Erfindung ist die Verwendung von Wirkstoffmischungen zur Herstellung von hypocholesterinämischen Mitteln mit der Maßgabe, daß man
(a) Phytostenole und/oder Phytostenolester und
(b) Potenzierungsmittel, ausgewählt aus der Gruppe, die gebildet wird von Chitosanen, Phytostenolsulfaten und/oder (Desoxy)Ribonucleinsäuren
einsetzt.

Überraschenderweise wurde gefunden, daß Chitosane, Phytostenolsulfate und/oder Desoxy- bzw. Ribonucleinsäuren, die selbst über keine oder nur sehr geringe hypochloesterinämischen Eigenschaften verfügen, als Potenzierungsmittel für Phytostenole und/oder Phytostenolester wirken, d.h. die Abnahme des Cholesteringehaltes im Serum in Gegenwart von Phytostenolen bzw. Phytostenolestem beschleunigen. In Gelatine verkapselt lassen sich sowohl die Phytostenole und/oder Phytostenolester als auch die Wirkstoffgemische zudem problemlos oral einnehmen.

### Phytostenole und Phytostenolester

Unter Phytostenolen (oder synonym Phytosterolen) sind pflanzliche Steroide zu verstehen, die nur am C-3 eine Hydroxylgruppe, sonst aber keine funktionellen Gruppen tragen. In der Regel besitzen die Phytostenole 27 bis 30 Kohlenstoffatome und eine Doppelbindung in 5/6, gegebenenfalls 7/8, 8/9 oder anderen Positionen. Durch Härtung können aus den ungesättigten Stenolen die entsprechenden gesättigten Stanole erhalten werden, die von der Erfindung mitumfaßt werden. Typische Beispiele für geeignete Phytostenole sind Ergostenole, Campestenole, Stigmastenole, Brassicastenole, vorzugsweise Sitostenole bzw. Sitostanole und insbesondere β-Sitostenole bzw. β-Sitostanole. Neben den genannten Phytostenolen werden vorzugsweise deren Ester eingesetzt Die Säurekomponente der Esters kann auf Carbonsäuren der Formel (I) zurückgehen,

**R**^{**1**}**CO-OH (I)**

in der R¹CO für einen aliphatischen, linearen oder verzweigten Acylrest mit 2 bis 22 Kohlenstoffatomen und 0 und/oder 1, 2 oder 3 Doppelbindungen steht. Typische Beispiele sind Essigsäure, Propionsäure, Buttersäure, Valeriansäure, Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise Kokos-, Palm-, Palmkernoder Talgfettsäure. Besonders bevorzugt ist der Einsatz von Estem des β-Sitostenols bzw. β-Sitostanols mit Fettsäuren mit 12 bis 18 Kohlenstoffatomen. Diese Ester können sowohl durch direkte Veresterung der Phytostenole mit den Fettsäuren oder aber durch Umesterung mit Fettsäureniedrigalkylestem oder Triglyceriden in Gegenwart geeigneter Katalysatoren, wie z.B. Natriumethylat oder speziell auch Enzyrnen hergestellt werden [vgl. **EP-A2 0195311** (Yoshikawa)].

### Chitosane

Chitosane, die die Komponente (b1) bilden, stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein **(II)** enthalten:

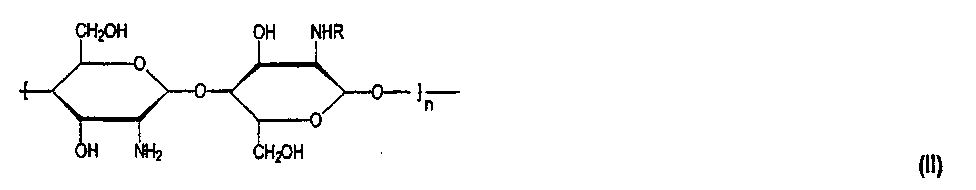

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332).** Übersichten zu diesem Thema sind auch beispielsweise von B.Gesslein et al. in **HAPPI 27, 57 (1990),** O.Skaugrud in **Drug Cosm.lnd. 148, 24 (1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden entweder niedermolekulare Chitosane mit einem durchschnittlichen Molekulargewicht von etwa 50.000 bis etwa 250.000 Dalton oder hochmolekulare Chitosane mit einem durchschnittlichen Molekulargewicht von etwa 500.000 bis etwa 2.000.000 eingesetzt. Entsprechende Verfahren sind beispielsweise aus **Makromol.Chem. 177, 3589 (1976)** oder der französischen Patentanmeldung **FR-A 2701266** bekannt. Besonders bevorzugt werden solche Typen eingesetzt, wie sie in den deutschen Patentanmeldungen **DE-A1 4442987** und **DE-A1 19537001** (Henkel) offenbart werden und die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE-C2 3713099** (L'Oréal) sowie der deutschen Patentanmeldung **DE-A1 19604180** (Henkel) beschrieben werden.

### Phytostenolsulfate

Phytostenolsulfate, die die Komponente (b2) bilden, stellen bekannte Stoffe dar, die beispielsweise durch Sulfatierung von Phytostenolen mit einem Komplex aus Schwefeltrioxid und Pyridin in Benzol hergestellt werden können [vgl. **J.Am.Chem.Soc. 63 1259 (1941)**]**.** Typische Beispiele sind die Sulfate von Ergostenolen, Campestenolen, Stigmastenolen und Sitostenolen. Die Phytostenolsulfate können als Alkali- und/oder Erdalkalimetallsalze, als Ammonium-, Alkylammonium-, Alkanolammonium- und/oder Glucammoniumsalze vorliegen. In der Regel werden sie in Form ihrer Natriumsalze eingesetzt.

### (Desoxy)Ribonucleinsäuren

Unter (Desoxy)Ribonucleinsäuren (DNA bzw. RNA), die die Komponente (b3) bilden, werden hochmolekulare, fadenförmige Polynuc-leotide verstanden, die sich von 2'-Desoxy-β-D-ribonucleosiden bzw. D-Ribonucleosiden ableiten, die ihrerseits wieder von äquivalenten Mengen einer Nucleobase und der Pentose 2-Desoxy-D-ribo-furanose bzw. D-Ribofuranose aufgebaut werden. Als Nucleobasen können die DNA bzw. RNA die Purinderivate Adenin und Guanin sowie die Pyrimidine Cytosin und Thymin bzw. Uracil enthalten. In den Nucleinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzelstrang-DNA bzw. - RNA. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton reichen. Im Sinne der Erfindung werden konzentrierte DNA bzw. RNA-Lösungen eingesetzt, die sich durch ein flüssigkristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 40.000 bis 1.000.000 Dalton aufweisen.

### Gewerbliche Anwendbarkeit

Die Wirkstoffmischungen der Erfindung können die Phytostenole und/oder Phytostenolester und die Potenzierungsmittel im Gewichtsverhältnis 99:1 bis 1:99, vorzugsweise 90:10 bis 10:90, insbesondere 70: 25 bis 25 : 75 und besonders bevorzugt 60 : 40 bis 40 : 60 enthalten, wobei allein sicherzustellen ist, daß mit der erfindungsgemäßen Verwendung eine zur Senkung des Cholesteringehaltes im Blut ausreichende Menge der Komponente (a) aufgenommen wird. In einer besonderen Ausführungsform der Erfindung werden die Wirkstoffmischungen in an sich bekannter Weise in Gelatine verkapselt, wobei man die Komponenten (a) und (b) jeweils in Mengen von 0,1 bis 50, vorzugsweise 1 bis 30, insbesondere 5 bis 25 und besonders bevorzugt 10 bis 15 Gew.-% - bezogen auf das Gewicht der Gelatinekapseln - einsetzt. Ein weiterer Aspekt der Erfindung betrifft die Erkenntnis, daß die Verkapselung der Phytostenole undloder Phytostenolester in Gelatine bereits eine vorteilhafte Ausführungsform für die orale Aufnahme der Wirkstoffe darstellt.

Eine weitere Verabreichungsform der Wirkstoffmischungen sind Zäpfchen, die rektal oder vaginal eingeführt werden können, und als Supositoriengrundmasse ebenfalls Gelatine, gegebenenfalls in Kombination mit Glycerin, oder aber synthetische Fette bzw. Wachse, Polyethylenglycole oder natürliche Bestandteile wie z.B. Kakaobutter enthalten können.

Daneben ist es möglich, die Mischungen in üblichen Nahrungsmitteln zu lösen bzw. zu dispergieren, als da beispielsweise sind : Salatöle, Dressings, Mayonnaisen, Margarinen, Butter, Fritierfette, Kakaoprodukte, Wurst und dergleichen.

### Beispiele

### Beispiele 1 bis 16, Vergleichsbeispiele V1 bis V4

Es wurden Gelatinekapseln (Gewicht ca. 1,5 g) mit einem Gehalt von 5 bzw. 10 Gew.-% β-Sitostanol bzw. β-Sitostanolester und gegebenenfalls 5 bzw. 10 Gew.-% der verschiedenen Potenzierungsmittel sowie 0,5 Gew.-% radioaktiv markiertem Cholesterin hergestellt. Zur Untersuchung der hypocholesterinämischen Wirkung ließ man männliche Ratten (Einzelgewicht ca. 200 g) über Nacht fasten. Am folgenden Tag wurde den Versuchstieren jeweils eine zerkleinerte Gelatinekapsel mit etwas kochsalzhaltigem Wasser über eine Magensonde eingeführt. Nach 3, 6, 12, 24 und 48 h wurde den Tieren Blut abgenommen und der Gehalt an radioaktivem Cholesterin bestimmt. Die Ergebnisse, die den Mittelwert der Messungen von 10 Versuchstieren darstellen, sind in den Tabellen 1 und 2 zusammengefaßt. Die Angaben zur Abnahme der Radioaktivität verstehen sich jeweils in Bezug auf eine Blindgruppe von Versuchstieren, denen lediglich Gelatinekapseln mit einem Gehalt von 20 Gew.-% Vitamin E und einer entsprechenden Menge radioaktiv markiertem Cholesterin verabreicht worden war. Die Mischungen 1 bis 16 sind erfindungsgemäß, die Mischungen V1 bis V4 dienen dem Vergleich.

**Tabelle 1**

| **Hypocholesterinämische Wirkung (Mengenangaben als Gew.-% bezogen auf Gelatinekapsel)** | | | | | | | | | | |
|---|---|---|---|---|---|---|---|---|---|---|
| **Zusammensetzung/Aktivltät** | **V1** | **1** | **2** | **3** | **4** | **V2** | **5** | **6** | **7** | **8** |
| β-Sitostanol | 5 | 5 | 5 | 5 | 5 | 10 | 10 | 10 | 10 | 10 |
| Chitosan | - | 5 | - | - | - | - | 5 | - | - | - |
| β-Sitostenolsulfat-Natriumsalz | - | - | 5 | - | - | - | - | 5 | - | - |
| Marine DNA (M = 150.000) | - | - - | - | 5 | - | - | - | - | 5 | - |

| **Radioaktivität *[%-rel]*** | | | | | | | | | | |
|---|---|---|---|---|---|---|---|---|---|---|
| ***- nach 3 h*** | 93 | 93 | 93 | 93 | 93 | 92 | 92 | 92 | 91 | 91 |
| ***- nach 6 h*** | 83 | 80 | 82 | 82 | 81 | 78 | 76 | 75 | 75 | 74 |
| ***- nach 12 h*** | 75 | 70 | 72 | 73 | 71 | 61 | 55 | 57 | 57 | 59 |
| ***- nach 24 h*** | 50 | 44 | 45 | 46 | 45 | 35 | 27 | 29 | 38 | 30 |
| ***- nach 48 h*** | 32 | 22 | 26 | 25 | 24 | 21 | 16 | 18 | 18 | 19 |

**Tabelle 2**

| **Hypocholesterinämische Wirkung (Mengenangaben als Gew.-% bezogen auf Gelatinekapsel)** | | | | | | | | | | |
|---|---|---|---|---|---|---|---|---|---|---|
| **Zusammensetzung/Aktivität** | **V3** | **9** | **10** | **11** | **12** | **V4** | **13** | **14** | **15** | **16** |
| Laurinsäure-β-sitostanolester | 5 | 5 | 5 | 5 | 5 | 10 | 10 | 10 | 10 | 10 |
| Succinyliertes Chitosan | - | 5 | - | - | - | - | 5 | - | - | - |
| Chitosan | - | - | 5 | - | - | - | - | 5 | - | - |
| β-Sitostenolsulfat-Natriumsalz | - | - | - | 5 | - | - | - | - | 5 | - |
| Marine DNA (M = 150.000) | - | - | - | - | 5 | - | - | - | - | 5 |

| ***Radioaktivität [%-rel]*** | | | | | | | | | | |
|---|---|---|---|---|---|---|---|---|---|---|
| ***- nach 3 h*** | 95 | 94 | 94 | 94 | 94 | 93 | 91 | 91 | 91 | 91 |
| ***- nach 6 h*** | 84 | 83 | 83 | 83 | 82 | 79 | 80 | 76 | 76 | 75 |
| ***- nach 12 h*** | 76 | 71 | 72 | 72 | 72 | 62 | 60 | 58 | 57 | 58 |
| ***- nach 24 h*** | 51 | 44 | 45 | 45 | 46 | 34 | 31 | 29 | 39 | 29 |
| ***- nach 48 h*** | 30 | 28 | 25 | 26 | 25 | 20 | 20 | 17 | 17 | 18 |

## Patentansprüche

1. Verwendung von Wirkstoffmischungen zur Herstellung von hypocholesterinämischen Mitteln, **dadurch gekennzeichnet, daß** man
(a) Phytostenole und/oder Phytostenolester und
(b) Potenzierungsmittel, ausgewählt aus der Gruppe, die gebildet wird von Chitosanen, Phytostenolsulfaten undloder (Desoxy)Ribonucleinsäuren
einsetzt.

2. Verwendung nach Anspruch 1, **dadurch gekennzeichnet, daß** man als Komponente (a) β-Sitostenol, β-Sitostanol oder deren Ester einsetzt.

3. Verwendung nach den Ansprüchen 1 und 2, **dadurch gekennzeichnet, daß** man als Komponente (a) Ester von β-Sitostenol bzw. β-Sitostanol mit Carbonsäuren der Formel (I) einsetzt,
**R**^{**1**}**CO-OH (I)**
in der R¹CO für einen aliphatischen, linearen oder verzweigten Acylrest mit 2 bis 22 Kohlenstoffatomen und 0 und/oder 1, 2 oder 3 Doppelbindungen steht.

4. Verwendung nach den Ansprüchen 1 bis 3, **dadurch gekennzeichnet, daß** man als Komponente (a) Ester von β-Sitostenol bzw. β-Sitostanol mit Fettsäuren mit 12 bis 18 Kohlenstoffatomen einsetzt.

5. Verwendung nach den Ansprüchen 1 bis 4, **dadurch gekennzeichnet, daß** man als Komponente (b1) Chitosane mit einem durchschnittlichen Molekulargewicht im Bereich von 50.000 bis 250.000 bzw. 500.000 bis 2.000.000 Dalton einsetzt.

6. Verwendung nach den Ansprüchen 1 bis 4, **dadurch gekennzeichnet, daß** man als Komponente (b1) succinylierte Chitosane einsetzt.

7. Verwendung nach den Ansprüchen 1 bis 6, **dadurch gekennzeichnet, daß** man als Komponente (b3) marine Desoxyribonucleinsäuren einsetzt, die ein Molekulargewicht im Bereich von 40.000 bis 1.000.000 Dalton aufweisen.

8. Verwendung nach den Ansprüchen 1 bis 7, **dadurch gekennzeichnet, daß** man die Komponenten (a) und (b) im Gewichtsverhältnis 99:1 bis 1:99 einsetzt.

9. Verwendung nach den Ansprüchen 1 bis 8, **dadurch gekennzeichnet, daß** man die Komponenten (a) und (b) in Gelatine verkapselt.

10. Verwendung nach Anspruch 9, **dadurch gekennzeichnet, daß** man die Komponenten (a) und (b) jeweils in Mengen von 0,1 bis 50 Gew.-% - bezogen auf das Gewicht der Gelatinekapseln - einsetzt.

## Claims

1. The use of active-substance mixtures for the production of hypocholesterolemic preparations, **characterized in that**
(a) phytostenols and/or phytostenol esters and
(b) potentiating agents selected from the group consisting of chitosans, phytostenol sulfates and/or (deoxy)ribonucleic acids
are used.

2. The use claimed in claim 1, **characterized in that** β-sitostenol, β-sitostanol or esters thereof are used as component (a).

3. The use claimed in claims 1 and 2, **characterized in that** esters of β-sitostenol or β-sitostanol with carboxylic acids corresponding to formula **(I)**:
**R**^{**1**}**CO-OH**
in which R¹CO is an aliphatic, linear or branched acyl group containing 2 to 22 carbon atoms and 0 and/or 1, 2 or 3 double bonds,
are used as component (a).

4. The use claimed in claims 1 to 3, **characterized in that** esters of β-sitostenol or β-sitostanol with fatty acids containing 12 to 18 carbon atoms are used as component (a).

5. The use claimed in claims 1 to 4, **characterized in that** chitosans with an average molecular weight of 50,000 to 250,000 or 500,000 to 2,000,000 dalton are used as component (b1).

6. The use claimed in claims 1 to 4, **characterized in that** succinylated chitosans are used as component (b1).

7. The use claimed in claims 1 to 6, **characterized in that** marine deoxyribonucleic acids with a molecular weight of 40,000 to 1,000,000 dalton are used as component (b3).

8. The use claimed in claims 1 to 7, **characterized in that** components (a) and (b) are used in a ratio by weight of 99:1 to 1:99.

9. The use claimed in claims 1 to 8, **characterized in that** components (a) and (b) are encapsulated in gelatine.

10. The use claimed in claim 9, **characterized in that** components (a) and (b) are each used in quantities of 0.1 to 50% by weight, based on the weight of the gelatine capsules.

## Revendications

1. Utilisation de mélanges de principes actifs en vue de la production d'agents hypocholestérolémiants,
**caractérisée en ce qu'**
on met en oeuvre
a) des phytosténols et/ou des esters de phytosténol, et
b) des agents de potentialisation choisis dans le groupe qui est formé des chitosanes, des sulfates de phytosténol et /ou des acides (désoxy)ribonucléiques.

2. Utilisation selon la revendication 1,
**caractérisée en ce qu'**
on met en oeuvre comme composant a) du β-sitosténol, du β-sitostanol ou leurs esters.

3. Utilisation selon les revendications 1 et 2,
**caractérisée en ce qu'**
on met en oeuvre comme composant a) des esters de β-sitosténol ou de β-sitostanol avec des acides carboxyliques de formula (I)
R¹-CO-OH (I)
dans laquelle R¹CO représente un reste acide acyle aliphatique linéaire ou ramifié ayant de 2 à 22 atomes de carbone, et 0 et/ou 1, 2 ou 3 double liaison.

4. Utilisation salon les revendications 1 à 3,
**caractérisée en ce qu'**
on met en oeuvre comme composant a) des esters de β-sitosténol ou de β-sitostanol avec des acides gras ayant de 12 à 18 atomes de carbone.

5. Utilisation selon des revendication 1 à 4,
**caractérisée en ce qu'**
on met en oeuvre comme composant (b1) des chitosanes ayant un poids moléculaire moyen dans la zone de 50.000 à 250.000 ou de 500.000 à 2.000.000 de Daltons.

6. Utilisation selon les revendications 1 à 4,
**caractérisée en ce qu'**
on met en oeuvre comme composant (b1) du chitosane succinylé.

7. Utilisation selon les revendications 1 à 6,
**caractérisée en ce qu'**
on met en oeuvre comme composant (b3) des acides désoxyribonucléiques marins qui possèdent un poids moléculaire dans la zone de 40.000 à 1.000 000 de Daltons.

8. Utilisation selon les revendications 1 à 7
**caractérisée en ce qu'**
on met en oeuvre les composants a) et b) dans un rapport en poids allant 99 : 1 à 1 : 99.

9. Utilisation selon les revendications 1 à 8,
**caractérisée en ce qu'**
on encapsule les composants (a) et (b) dans de la gélatine.

10. Utilisation selon la revendication 9,
**caractérisée en ce qu'**
on met en oeuvre les composants (a) et (b) respectivement en quantité allant de 0,1 à 50 % en poids, rapporté au poids des capsules de gélatine.
